Europäisches Patentamt

European Patent Office

Office européen des brevets

(11) Publication number : **0 379 793 B1**

# EUROPEAN PATENT SPECIFICATION

(45) Date of publication of patent specification :
10.03.93 Bulletin 93/10

(51) Int. Cl.$^5$ : **C07C 19/08**

(21) Application number : **89312344.8**

(22) Date of filing : **28.11.89**

(54) Process for the preparation of 1,1,1,2-tetrafluorethane.

(30) Priority : **07.12.88 GB 8828544**

(43) Date of publication of application :
**01.08.90 Bulletin 90/31**

(45) Publication of the grant of the patent :
**10.03.93 Bulletin 93/10**

(84) Designated Contracting States :
**DE FR GB IT NL**

(56) References cited :
**EP-A- 0 349 115**
**US-A- 4 766 260**

(73) Proprietor : **IMPERIAL CHEMICAL INDUSTRIES PLC**
**Imperial Chemical House, Millbank**
**London SW1P 3JF (GB)**

(72) Inventor : **McCulloch, Archibald**
**Barrymore Marbury Road**
**Comberbach Northwich Cheshire (GB)**
Inventor : **Powell, Richard Llewellyn**
**9 Sadlers Wells**
**Bunbury Tarporley Cheshire CW6 9NU (GB)**
Inventor : **Robinson, Gareth Charles**
**41 Hatherton Way**
**Northgate Village Chester CH2 2AE (GB)**
Inventor : **Steven, James Hugo**
**5 Stockham Close**
**Runcorn Cheshire (GB)**
Inventor : **Young, Brian David**
**11 Carisbrook Drive**
**Winsford Cheshire (GB)**

(74) Representative : **Stephenson, Kenneth et al**
**ICI Group Patents Services Dept. PO Box 6**
**Shire Park Bessemer Road**
**Welwyn Garden City, Herts, AL7 1HD (GB)**

## Description

This invention relates to a chemical process and more particularly to a process for the manufacture of 1,1,1,2-tetrafluoroethane.

Several methods have been proposed for the manufacture of 1,1,1,2-tetrafluoroethane which is a useful refrigerant, aerosol propellant, blowing agent and solvent. Thus, our United Kingdom Patent No 1578933 describes a process for making tetrafluoroethanes by hydrogenating dichlorotetrafluoroethanes at elevated temperatures, for example temperatures in the range 200-450°C. Also, our United Kingdom Patent No 2004539 describes the manufacture of 1,1,1,2-tetrafluoroethane by reacting trifluoroethylene in the vapour phase with hydrogen fluoride in the presence of chromium oxide, suitable reaction temperatures being in the range 200-500°C. Both of these processes are expensive to operate.

It has now been found that 1,1,1,2-tetrafluoroethane can be prepared in high yield and high selectivity by the two stage process hereinafter described.

The present invention provides a method for the preparation of 1,1,1,2-tetrafluoroethane which comprises the steps of:

(i) contacting an antimony halide fluorination catalyst at an elevated temperature with tetrachloroethylene or with a mixture of or reaction product of trichloroethylene and chlorine whereby to form 1-chloro-1,2,2,2-tetrafluoroethane, and

(ii) hydrogenating the 1-chloro-1,2,2,2-tetrafluoroethane at an elevated temperature in the presence of a hydrogenation catalyst.

Antimony halide fluorination catalysts have been fully described in the prior art and include antimony fluorides, mixtures thereof with antimony chlorides and antimony fluorochlorides, at least part of the antimony in the catalyst being in a pentavalent state. If desired, the catalyst may be prepared in situ from antimony pentachloride or mixtures thereof with antimony trichloride by including hydrogen fluoride in the reaction mixture.

The first stage of the method of the invention may be conveniently operated by contacting tetrachloroethylene in the liquid phase with the antimony halide fluorination catalyst, optionally in conjunction with hydrogen fluoride, at an elevated temperature, for example a temperature in the range from about 50°C to about 150°C and at autogenous pressure. Alternatively, a mixture of trichloroethylene and chlorine or a reaction product thereof, that is to say pentachloroethane, may be employed in place of tetrachloroethylene.

The second stage of the method of the invention may be suitably carried out in the vapour phase at a temperature in the range from about 100°C to about 450°C, for example 150 to 300°C, the 1-chloro-1,2,2,2-tetrafluoroethane produced in the first stage being contacted with hydrogen and a hydrogenation catalyst. Atmospheric or superatmospheric pressures may be employed. Suitable hydrogenation catalysts include metals of Group VIIIA of the Periodic Table or oxides or salts thereof, palladium being especially useful. The catalyst may be carried on a conventional support, for example alumina (especially a high surface area alumina such as an eta alumina), aluminium fluoride or activated carbon. The catalyst may be dispersed on the support by conventional means, for example by using a palladium halide salt. Palladium loadings of 0.5 to 20% (as the metal) are typical. The catalyst may be in the form of a fluid bed or a packed bed.

Conventional techniques may be employed at each stage for separating the desired product from unchanged starting materials and/or by-products. Thus 1-chloro-1,2,2,2-tetrafluoroethane will usually be isolated from the first stage reaction product before hydrogenation.

The invention is illustrated but not limited by the following Examples.

### Example 1

(i) A 1 litre Hastelloy autoclave pot was charged with $SbF_5$ (957 g, 4.43 mol) attached to the autoclave lid fitted with pressure gauge, vapour sample point. thermopocket and bursting disc) and evacuated. The autoclave pot was then cooled to 9°C and pentachloroethane (300 g, 1.49 mol) was sucked into the evacuated pot. The pot was re-evacuated. The autoclave pot was then heated with agitation to a temperature of 130°C. A pressure of 10 bar was generated. A vapour sample was taken and shown by GC to contain $CF_3CHClF$ (91.3%), $CF_3CHF_2$ (2.1%), $CF_3CHCl_2$ (1.7%) and $CF_2ClCF_2Cl/CF_3CFCl_2$ (3.5%).

$CF_3CHClF$ was isolated from the reaction product by conventional means.

(ii) 50 ml of a 5% Pd on alumina catalyst was dried and reduced. Hydrogen and $CF_3CHClF$ were passed through the reactor bed at a mole ratio of 1:1 to give a contact time of 14 seconds. With a bed temperature of 235°C, the conversion was 62.18% with a selectivity to $CF_3CH_2F$ of 93.80%. Other products obtained were lights (2.93%; methane, ethane and $CF_3CH_3$) and $CF_3CH_2Cl$ (1.35%). At 260°C the conversion was 75.90% with a selectiviy to $CF_3CH_2F$ of 89.60%. Products are lights (2.4%), $CF_3CH_2F$ (68.1%) and $CF_3CH_2Cl$ (2.4%).

Example 2

(i) An 800 ml Inconel autoclave pot, fitted with a PTFE liner, was charged with SbCl₅ (363 g, 1.21 mol) and attached to the autoclave lid which was fitted with a pressure control valve, thermopocket, vapour sample point, HF feed point, organic feed point and chlorine feed point. An initial charge of pentachloroethane (80.2 g, 0.4 mol) was fed into the autoclave pot followed by an initial charge of anhydrous liquid hydrogen fluoride (105 g, 5.25 mol). The contents of the autoclave pot were heated with agitation, to temperatures between 110°C and 120°C, the pressure being controlled at 24.0 bar by the pressure control valve. The liquid level in the autoclave pot was maintained by continuously pumping HF and pentachloroethane into the pot. Chlorine vapour was periodically fed into the pot to maintain the antimony fluorochloride catalyst in a high oxidation state. GC analysis of the vapours escaping $\underline{via}$ the pressure control valve showed that they contained $CF_3CHClF$(13.8%), $CF_3CHCl_2$(71.3%), $CF_2ClCHCl_2$(4.8%),, $CF_2ClCHClF$(0.3%), $CF_2ClCF_2Cl$ (7.1%), $CF_2ClCFCl_2$(1.1%) and $CF_3CH_2Cl$ (1.2%).

$CF_3CHClF$ was isolated from the reaction product by conventional means.

(ii) 50g of 1% Pd on aluminium fluoride powder was dried and reduced in the reactor. $CF_3CHClF$ and hydrogen were mixed (1:1 mole ratio) and heated to the reactor temperature. The off-gases were analysed by GC.

Typical analyses

| Bed temp | Yield (v/v%) | | | | |
|---|---|---|---|---|---|
| °C | methane | ethane | $CF_3CH_3$ | $CF_3CH_2F$ | $CF_3CH_2Cl$ |
| 202 | 0.11 | 0.78 | 0.2 | 22.1 | 0.27 |
| 211 | 0.19 | 1.24 | 0.36 | 30.71 | 0.49 |
| 221 | 0.34 | 1.93 | 0.5 | 40.5 | 0.84 |
| 250 | 2.27 | 5.14 | 0.74 | 61.58 | 1.05 |

**Claims**

1. A method for the preparation of 1,1,1,2-tetrafluoroethane which comprises the steps of:
   (i) contacting an antimony halide fluorination catalyst at an elevated temperature with tetrachloroethylene or with a mixture of or reaction product of trichloroethylene and chlorine whereby to form 1-chloro-1,2,2,2-tetrafluoroethane, and
   (ii) hydrogenating the 1-chloro-1,2,2,2-tetrafluoroethane at an elevated temperature in the presence of a hydrogenation catalyst.

2. A method according to Claim 1 wherein step (i) comprises contacting pentachloroethane with the fluorination catalyst at a temperature in the range from 50 to 150°C.

3. A method according to Claim 1 or Claim 2 wherein the hydrogenation catalyst employed in step (ii) comprises palladium on an alumina, aluminium fluoride or activated carbon support.

**Patentansprüche**

1. Verfahren zur Herstellung von 1,1,1,2-Tetrafluoroethan, welches die folgenden Schritte umfaßt:
   (i) Inkontaktbringen eines Antimonhalogenid-Fluorierungskatalysators bei einer erhöhten Temperatur mit Tetrachloröethylen oder einer Mischung von oder dem Reaktionsprodukt von Trichloroethylen und Chlor, um 1-Chloro-1,2,2,2-Tetrafluoroethan zu bilden, und
   (ii) Hydrieren des 1-Chloro-1,2,2,2-Tetrafluoroethan bei einer erhöhten Temperatur in Gegenwart eines Hydrierungskatalysators.

2. Verfahren nach Anspruch 1, dadurch gekennzeichnet, daß Schritt (i) das Inkontaktbringen von Pentachloroethan mit dem Fluorierungskatalysator bei einer Temperatur im Bereich von 50 bis 150°C umfaßt.

3.  Verfahren nach Anspruch 1 oder 2, dadurch gekennzeichnet, daß der in Schritt (ii) verwendete Hydrierungskatalysator Palladium auf einem Aluminiumoxid-, Aluminiumfluorid- oder Aktivkohlenstoff-Träger umfaßt.


**Revendications**

1.  Procédé de production de 1,1,1,2-tétrafluoréthane, qui comprend les étapes consistant :
    (i) à faire entrer un catalyseur de fluoration constitué d'halogénure d'antimoine, à une température élevée, en contact avec du tétrachloréthylène ou avec un mélange de trichloréthylène et de chlore ou le produit de réaction du trichloréthylène et du chlore de manière à former du 1-chloro-1,2,2,2-tétrafluoréthane, et
    (ii) à hydrogéner le 1-chloro-1,2,2,2-tétrafluoréthane à une température élevée en présence d'un catalyseur d'hydrogénation.

2.  Procédé suivant la revendication 1, dans lequel l'étape (i) consiste à faire entrer du pentachloréthane en contact avec le catalyseur de fluoration à une température comprise dans la plage de 50 à 150°C.

3.  Procédé suivant la revendication 1 ou la revendication 2, dans lequel le catalyseur d'hydrogénation utilisé dans l'étape (ii) comprend du palladium fixé sur un support d'alumine, de fluorure d'aluminium ou de carbone activé.